# EUROPEAN PATENT SPECIFICATION

(11) **EP 1 003 832 B1**
(45) Date of publication and mention of the grant of the patent: **04.02.2004**
(21) Application number: 98939244.4
(22) Date of filing: 05.08.1998
(51) Int. Cl.: C12M 1/28

(54) **METHOD AND APPARATUS FOR SEPARATING PARTICULATE MATTER FROM A LIQUID SPECIMEN**
ANLAGE UND VERFAHREN ZUM TRENNEN VON FESTSTOFFEN AUS EINER FLÜSSIGENPROBE
PROCEDE ET DISPOSITIF SERVANT A SEPARER DES PARTICULES DE MATIERE DEPUIS UN SPECIMEN DE LIQUIDE

(30) Priority: 05.08.1997 US 54799 P; 04.11.1997 US 963873
(43) Date of publication of application: 31.05.2000
(73) Proprietor: LAMINA, INC., Arlington, VA 22209 (US)
(72) Inventor: GUIRGUIS, Raouf, A., Vienna, VA 22182 (US); EL-AMIN, Marianna, Bethesda, MD 20817 (US); SAMAAN, Nashed, Germantown, MD 20876 (US)
(74) Representative: Evens, Paul Jonathan
(86) International application number: PCT/US1998/016349
(87) International publication number: WO 1999/007823

(56) References cited:
- EP-A- 0 448 837
- EP-A- 0 471 570
- EP-A- 0 483 506
- WO-A-94/03103
- FR-A- 2 691 977
- US-A- 5 422 273
- US-A- 5 624 554

## Description

### TECHNICAL FIELD OF THE INVENTION

The present invention is directed to an apparatus and method for collecting a uniform monolayer of particulate matter. In particular, the present invention is directed to an apparatus and manual or semi-automatic method for collecting a uniform monolayer of cells from biological fluids and preparing the monolayer of cells for use in cytological protocols.

### BACKGROUND OF THE INVENTION

In a wide variety of technologies, the ability and/or facility for separating matter, typically particulate matter, from a fluid is a critical component in the ability to test for the presence of substances in the fluid. Too often, interference associated with sample preparation obscures the target cells to such a degree that the process is not sufficiently reliable, or too costly.

Such a scenario applies to many other fields which involve detection and/or diagnosis, including environmental testing, radiation research, cancer screening, cytological examination, microbiological testing, and hazardous waste contamination, to name just a few.

In all of these endeavors, limiting factors in the sample preparation protocol include adequately separating particulate matter from its fluid carrier (e.g., physiological fluid, biological fluid and environmental fluid), and easily and efficiently collecting and concentrating the particulate matter in a form readily accessible for microscopic examination.

In the case of cytological examination, a sample of cells is obtained from a patient. Typically, this is done by scraping or swabbing an area, as in the case of cervical samples, or by collecting body fluids, such as those obtained from the chest cavity, bladder, or spinal canal, or by fine needle aspiration. In a conventional manual cytological examination, particulate matter including cells and debris in the fluid are transferred onto a glass slide by smearing and subsequently air-dried. Smearing results in non-uniform densities and uneven distributions of cells and debris that often obscure the target cells. Air drying causes cell distortion and further impedes accurate examination.

It has been found that prompt processing of urine to obtain fresh ensures the accuracy of quantitative culture results, urinalysis and microscopy. Fresh cells tend to stick to a glass slide much better than cells from preserved urine, allowing for smoother cell spread onto the glass body. Delays in processing, negligent care in either inpatient or outpatient settings and lack of refrigeration may lead to non-optimal slide preparation. One known solution to the delay problem is the use of chemical preservatives with the urine. The presence of liquid preservatives, however, in the urine specimen raises the specific gravity of the specimen to unmeasurable levels and may limit the potential usefulness of the urine for various types of traditional quantitative analysis, such as slide microscopy.

Diagnostic microbiology and/or cytology, particularly in the area of clinical pathology, bases diagnoses on a microscopic examination of cells and other microscopic analyses. The accuracy of the diagnosis and the preparation of optimally interpretable specimens typically depends upon adequate sample preparation. New methodologies such as immunocytochemistry and image analysis require preparations that are reproducible, fast, biohazard-free and inexpensive. Conventional cell preparation techniques fail to adequately address the issues of non-uniform cell densities, uneven cell distribution and air drying artifacts.

Conventionally, body fluid samples are collected for cytological examinations using containers that contain a preservative solution for preserving the cytology specimen during shipment from the collection site to the cytology laboratory. Furthermore, cytology specimens collected from the body cavities using a swab, smear, flush or brush are also preserved in containers with fixatives (e.g., alcohol or acetone fixatives) prior to transferring cells onto the slide or membrane for staining or examination.

It is desirable to provide a urine or other biological fluid specimen container that would allow liquid biological specimens to be tested without removing the lid of the urine or biological fluid container. However, none of the prior art solves the problems of transferring cells in a monolayer to a slide for examination without submerging portions of the device in the sample (and increasing the risk of contamination), consistently and repeatedly forming a high quality monolayer on the microscope slide, and processing the sample so that the fluid from which the cells were taken is preserved.

A number of methods, apparatuses, and structures for dispersing cells in the fluid are known. For example, U.S. Patent 5,143,627 opens the sample container, inserts a dispersing element into the liquid suspension, and rotates the dispersing element for several minutes. In another example, the so-called "Saccomanno method" is used to process sputum, a process that is time consuming and involves a large number of processing steps.

Cytological test methods and instruments to quantitatively measure and to collect cells are known according to European Patent No. 0 448 837. This patent discloses a method and apparatus for the controlled instrumentation processing of cells and other particles with a filter device that measures a parameter of the flow through the filter device of a fluid carrying the particles. A measure of the change of the fluid flow through the filter device yields desired information for quantifying the particles and for quantifying the obstruction of the filter device by the particles. The method and apparatus according to this patent typically operate automatically.

Another limiting factor in optimally preparing the particulate matter for microscopic examination involves the solution and/or solutions for fixing the particulate matter to a microscope slide or the like.

Cytologic specimens, which constitute the examinable form of the cytologic material, may be prepared by well-understood smear or fluid techniques. Because there may be a considerable lapse of time before these specimens are further processed by staining, applying a cover slip, and so forth, however, it is important to apply a fixative to the cytologic material as a means of preserving and fixing the cells.

Properly fixing (i.e., preserving) cytologic material such as cells, cell aggregates and small tissue fragments derived from cytologic collections of human or animal tissue is a prerequisite to the accurate diagnosis of disease, especially cancer. Cytologic material must be fixed as soon as possible after obtaining the material to prevent cell distortion.

Air-dried and tetrachrome-dye stained cytologic specimens, although popular abroad, are not generally used in the United States. Rather, wet fixation, either by the immersion of slides into an alcohol solution, by saturation of slides with a spray fixative or by directly discharging cytologic material into an alcohol solution, is a known method of cell fixation. Cell fixation is a prerequisite for interpretable Papanicolaou, Hematoxylin and Eosin or other stained cytologic specimen slides.

Generally, alcohol solutions, with or without other additives such as polyethylene glycol, ranging from 50 % to 95% (v/v: methanol, ethanol, isopropanol) are known solutions for use in wet fixation. When alcohol solutions greater than 50% (v/v) are used for collecting and fixing fluids high in protein, however, a protein sediment forms which subsequently hardens. Protein sedimentation makes the fixed cytologic material difficult to transfer to glass slides for examination, regardless of whether the transfer is done by direct application to the glass slide, by cytofiltration through a small pore filter, or by cytocentrifugtion onto glass slides coated with an adhesive such as chrome aluminum gelatin.

For over a century, tissue fixative compositions used to preserve and prepare tissue for analytical evaluation have been based on formaldehyde. The standard composition employed for tissue preservation and the preparation of thin-cut tissue for microscopic examination is Formalin. Formalin is a 3 to 10 percent solution of formaldehyde in water, usually containing about 15 percent methyl alcohol. Alcohol improves the preservative properties of the solution. Despite numerous disadvantages, most notably high toxicity and irritant properties, Formalin remains the fixative of choice in typical laboratory applications owing to its rapid reaction with exposed tissue surfaces and consequent maximized cellular preservation. Methanol may adversely affect the texture of the tissue, rendering it too brittle or, more usually, too soft for ease in cutting for slide preparation. It also may produce pigmented artifacts or impurities that interfere with staining. Formalin containing methanol nevertheless provides preserved tissue that can be satisfactorily sectioned and stained for microscopic examination.

Histologists have long endeavored to develop effective immunohistochemical fixatives and morphologic fixatives. Moreover it is desirable to preserve morphologic detail preserve tissue antigens to permit immunohistochemical detection and localization of antigens in tissue.

Such fixatives render protein insoluble. For example, formaldehyde may be used as a crosslinking agent forming covalent bonds between the aldehyde groups and specific amino acids to stabilize protein structure and transform the cell cytoplasm into a gel which prohibits movement of autolytic enzymes. Alternately, alcohol may be used as a fixative to precipitate protein through denaturation.

Preferably, a fixative should retard autolysis and putrefaction and preserve morphologic detail and antigenicity. Unfortunately, an effective morphologic fixative is not necessarily an effective immunohistochemical fixative.

In contrast to the conventional techniques, the solid matter preparation techniques of the present invention address the issues of non-uniform matter densities, uneven matter distribution, and sample loss and contamination due to the number of steps involved in the sample preparation. Thus, preparations according to the present invention result in an even distribution of solids that have superior morphology, improved visualization, and are readily positioned and available for light absorbance analysis without the need to further manipulate or prepare the sample.

In accordance with the present invention, various aspects thereof are defined in the appendent indenpendent claims to which reference should now be made. Various embodiments of the present invention are defined in the appendent dependent claims.

The present invention relates to an apparatus and method for collecting matter for detection, analysis, quantification, and/or visualization. The devices and methods of the present invention are particularly suitable for separating particulate matter from biological, physiological, and environmental fluids and presenting the particulate matter in an improved manner for cytological examination.

A preferred embodiment of the present invention relates to an apparatus and method for collecting a uniform layer of cells from urine or other biological fluid specimen in a cytology collection apparatus or assay module, and for transferring the uniform layer of particulate matter to a slide.

The devices and methods of the present invention may be configured into a handheld manual system or structure, or a partially automated system or structure.

Such an apparatus according to the present invention overcomes the problems associated with conventional equipment for collecting cells and other particles for cytology by providing a mechanism of relatively simple structure and operation that separates particles from a liquid solution, collects an approximately known quantity of the cells in a monolayer, and transfers the collected cells to a microscope slide. In some embodiments of the present invention, no element of the apparatus is placed in the liquid sample, thus preventing unnecessary contamination of the sample. Moreover, in some embodiments of the present invention, the container holding the sample is not opened in the course of collecting and transferring the cells, thus eliminating the possibility of sample contamination during testing.

In all embodiments of the present invention, a monolayer of the particulate matter, e.g., cells, in the sample is collected on a filter by passing two branches of a fluid flow through and around the filter. Such a filter is known from U.S. Patent Numbers 5,301,685 and 5,471,994, which correspond to World Patent Number 94/03103.

The patient or medical person handling the collection may seal a separate container. The collection of the cells according to the present invention allows a uniform cell slide to be obtained without contamination of the cells by preservatives, workers or outside materials. The transfer from collection container to the cytology collection apparatus may be carried out without pouring or pipetting the collected specimen.

The present invention is directed to a cell collection and distribution apparatus that can be disassembled to allow face to face transfer of cells from the device to a slide for microscope examination. The present invention provides an improved apparatus and method for collecting a monolayer of cells that can be transferred to a microscope slide. The effectiveness of transferring the monolayer cells from the filter to a microscope slide has proven to be very high without differential cell loss. Microscopic examination shows that the cell distribution is the same on the slide as on the filter.

The devices of the present invention obviate the need for a trained technician to properly prepare a sample substrate. Thus, time, expense, and expertise are eliminated or reduced as critical factors in sample preparation protocols.

The devices and methods of the present invention also provide advantages in sample preparation because they are suitable for use with fresh, untreated cells, unmodified cells, and are particularly designed to provide a thin, uniform layer of solid matter (up to approximately 40 microns or more). This invention is particularly useful for collecting cells for a Pap smear.

The apparatuses and methods of the present invention have many advantages for conventional microbiology and hematology. The collected cells are in a predetermined area that is easily accessible to a radiant light source and to a wavelength absorbance meter. Because cells are concentrated in a single layer, they are almost always in one focal plane, thus eliminating or reducing interference by other particles and virtually eliminating technician time and expertise in establishing a proper reading. The minimal matter overlap achieved by the present invention ensures that all matter can be easily examined with little chance for critical solids to be obscured by clumps of overlapping solids or debris. Certain embodiments of the apparatuses of the present invention may be used in combination with other automated devices to detect and analyze any solid matter in a given population. They also permit a detailed analysis of the chemical composition of the matter.

The present invention also includes an improved apparatus and method for processing a fluid containing particulate matter. The apparatus and method include dispersing particulate matter in the sample, preferably by rotating the sample container around a fixed agitator or by rotating the agitator inside a fixed sample container. The present invention agitates the sample within the container to ensure break-up of large particulate matter, e.g., mucoid bodies in the case of sputum samples, and the even distribution of cells throughout the fluid. Agitation may occur as the result of relative motion between components of the sample container, non-uniform motion of the sample container, and/or inertial reaction forces applied to the sample by the container.

According to a preferred embodiment of the present invention structures and means are provided for rotating an agitator in relation to the container and/or the sample in the container. As described in more detail below, a preferred embodiment according to the present invention may include a cover within a cover, wherein the agitator is fixed to a freely rotatable outer cover and an inner cover is secured with respect to a stationary sample container. Such relative motion moves the agitator in relation to the sample, and disperses particulate matter in the fluid.

Further, providing a container cover that has a portion that is rotatable permits particulate matter stirring or dispersion without inserting a stirring mechanism into the sample, thus eliminating a source of contamination that plaques devices that are presently commercially available. In preferred embodiments of the present invention, the covering on the sample container may include a hollow tube, with or without a rotatable dispersing element, for withdrawing the sample from the container.

In a preferred embodiment of the invention, the cover comprises a first portion that fixedly engages the container and a second portion that may be rotatable in relation to the container. As used herein, rotatable in relation to the container refers to the relative movement of the first portion and the second portion; the first portion may be fixed and the second portion moveable, or the first portion may be moveable and the second portion fixed. In a most preferred embodiment, the second or inner portion of the cover is stationary and the first or outer portion is rotatable. In a preferred embodiment of the invention, the agitator is engaged by or fixed to the second portion of the cover.

An apparatus according to a preferred embodiment of the present invention may be configured to support, engage, and rotate a portion of a collection container so that the sample is mixed according to the present invention. An exemplary collection container includes a container or cup suitable for collecting and holding a specimen sample, a cap having a first position that is not rotatable in relation to the container and a second position that is rotatable in relation to the container, and an agitator engaged by or fixed to a portion of the cover and extending into the container. As used herein, configured to support, engage, and rotate refers to various configurations that may be adapted to perform the specific function. For example, an apparatus according to the invention may include a container support for positioning at least one sample container and rotating the container per se, and a sleeve or clamp for engaging and fixing a portion of the cap that communicates with an agitating element. Alternatively, the support may hold the container in a fixed position and a pulley, sleeve, or clamp may engage and rotate the portion of the cap that is fixed with respect to an agitator. In a preferred embodiment of the invention, a sleeve engages an inner portion of the cap, and holds the inner portion of the cap in a stationary position in relation to an outer portion of the cap.

Configurations or structures that engage a portion of the cap or the container typically include any member that positions, fixes and/or moves either that portion of the cap or the container. Exemplary members include, but are not limited to, a sleeve, one or more belts, one or more pulleys, one or more resilient bands, and the like.

The present invention is also a device for processing a fluid into one or more components, typically by removing particulate matter from the fluid. The present invention is directed to apparatuses and methods for collecting fluids, such as biological, physiological, or environmental fluids, and removing particulate matter from the fluid, without centrifugation, and diagnosing and testing the matter. In a preferred embodiment of the invention, particulate matter is collected on a collection site. In a most preferred embodiment of the invention, the particulate matter is collected in a monolayer and in a pre-determined spatial arrangement.

While a cytology collection apparatus according to the invention can be used for any biological fluid, it is particularly useful for preparing testing samples from urine and its associated cells for Pap smears. It is intended that the type of matter being processed should not limit the invention. In a most preferred embodiment of the invention, the fluid is urine and particulate matter is a cell. The particulate matter processing apparatus of the present invention also permits isolation and collection of fresh cells and/or microorganisms from biological fluids to perform DNA probe and chromosomal analysis once the proper buffer hemolyzes the cells.

In the case of cervical examinations, a scraping of the cervix is taken with a long-handled brush or broom. The handle is then shortened, such as by breaking or telescopic movement, and the brush is inserted into a specimen container. Conventionally, the container must be opened to remove the brush at the time of testing. Such a process increases the likelihood of contamination because the cover of the sample container must be opened, the brush typically retains cells if the testing is not performed soon after cell collection, and the operator must come into contact with the sample.

According to a preferred embodiment of the present invention, these problems are avoided by providing a system in which the brush not only remains in the collection container, but can also be used to disperse the collected cells during agitation. Further, the apparatus of the present invention is a closed system; once the apparatus is closed, it does not need to be opened in order to process any cells collected on the brush.

Further, providing a container cover that has a portion that is rotatable permits particulate matter stirring or dispersion without inserting a stirring mechanism into the sample, thus eliminating a source of contamination that plaques devices that are presently commercially available.

The present invention is also directed to a cytology collection and testing kit containing a cytology collection apparatus, replacement filters, replacement disposables, and/or other components, ingredients of a fixative composition as described below. The cytology collection kit may also include replacement filters, replacement disposables, and/or other components, ingredients or solutions typically used during cytological examinations. The kit might also include washing, fixative, and/or buffer solutions. A cervical kit may include a brush or broom, and a fluid suitable for storing the used brush until particulate matter on the brush can be processed through the filter assembly.

A preferred embodiment of the present invention is also directed to a tissue fixative composition for use in histopathological applications which rapidly penetrates tissue surfaces for maximum cellular preservation, leaves minimal pigmented artifacts, and permits accurate staining. The present invention is also directed to providing a cytologic and histologic fixative formulation that fixes and preserves cells, cell aggregates and small tissue fragments in a liquid suspension.

The present invention is also directed to providing a fixative formulation that retains tissue samples that are incidentally collected along with cytologic material for further histological processing.

The present invention is also directed to providing a fixative formulation that allows shipments of the liquid suspension of cells, cell aggregates and tissue fragments under conditions typically encountered in postal carriage, permitting remote users without available cytologists, cytotechnologists, physicians or other personnel experienced in the preparation of cytologic samples to fix a cytologic specimen for later processing, and whereby technically satisfactory cytologic sample slides may be produced therewith.

The invention may concern a method of preparing tissue for cutting, staining and /or microscopic evaluation wherein specimen tissue prior to dehydration is subjected to preservation with a storage stable tissue fixative solution of the present invention.

A unique cytologic and histologic fixative formulation and methods for using that formulation are disclosed. The formulation fixes and preserves individual cells, aggregates of cells and small fragments of tissue in a liquid suspension; minimizes protein precipitation in the liquid suspension; selectively eliminates or reduces red blood cell contamination of cytologic material and cytologic specimen slides; retains tissue samples that are incidentally collected along with cytologic material for further histologic processing; and allows shipment of cytologic material under conditions typically encountered in postal carriage, permitting remote users without available cytologists, cytotechnologists or other personnel experienced in the preparation of cytologic samples to have technically satisfactory cytologic sample slides.

According to another arrangement the matter collection apparatus may also include additional modules, removable or integrated, for treating the fluid. For example, the fluid may be treated with a matter collection module, in combination with a debris removal module, a chromatography module, and assay module, or combinations of these and other devices. These and other modules or treatment protocols provide features that may be desirable to incorporate into a sample preparation apparatus according to the invention.

The devices and methods of the present invention have many advantages for conventional cytology. The cells are in a predetermined area allowing for significant timesaving when screening the slide. Such problems as cells lying outside the coverslip or on the frosted end are eliminated. Because cells are lying in a single layer, they are almost always in a one focal plane when using a 10X objective - the objective most often used for the lower power screening of a slide. Even with a 40X objective, most cells are in focus. This eliminates frequent refocusing and saves time.

The accompanying drawings show illustrative embodiments of the invention from which these and other of the objectives, novel features and advantages will be readily apparent.

### DESCRIPTION OF THE DRAWINGS

Figure 1 is a cross section view of a first preferred embodiment of the present invention.
Figure 2 is a cross section view of the particulate matter separation chamber according to the present invention.
Figure 3 is a cross section view of the fluid flow paths through the particulate matter separation chamber.
Figure 4A is a top view of the base and well assembly, forming the bottom portion of the particulate matter separation chamber.
Figure 4B is a top view of the bottom portion of the particulate matter separation chamber, and illustrates a clock face surface modification of the well.
Figure 4C is a top view of the bottom portion of the particulate matter separation housing, and illustrates cross hatch face surface modification of the well.
Figure 5 is a cross section view of a disassembled base, hollow tube, and container.
Figure 6 is a bottom view of the top portion of the particulate matter separation housing.
Figure 7 is a cross section view of the bottom portion of the particulate matter separation housing, and shows the optional channel and optional flap.
Figure 8 is a cross section view of the bottom portion of the particulate matter separation housing, and shows the optional channel and optional O-ring.
Figure 9 is a cross section view of the bottom portion of the particulate matter separation housing, and shows the optional channel and optional flap.
Figure 10 is a cross section view of a second preferred embodiment of the present invention.
Figure 11 is a cross section view of a third preferred embodiment of the present invention.
Figure 12 is a combination of bottom and side views of a filter arrangement according to a preferred embodiment of the invention.
Figure 13 is a cross section view of an apparatus used in a semi-automatic method according to a preferred embodiment of the present invention.
Figure 14 is a schematic illustration of the apparatus shown in Figure 13 in a first position.
Figure 15 is a schematic illustration of the apparatus shown in Figure 13 in a second position.

### DETAILED DESCRIPTION OF THE INVENTION

The present invention is a specimen container that includes a particulate matter separation chamber or module in fluid communication with a specimen container.

The present invention is also a device for processing a fluid into one or more components, typically by removing particulate matter from the fluid.

The present invention also includes devices and methods for collecting fluids, such as biological, physiological, or environmental fluids, removing the desired particulate matter from the fluid, without centrifugation, and diagnosing and testing the particulate matter. In a preferred embodiment of the invention, particulate matter is collected on a collection site. In a most preferred embodiment of the invention, the particulate matter is collected in a monolayer and in a pre-determined spatial arrangement.

The present invention also includes an improved apparatus and method for processing a fluid containing particulate matter. The apparatus and method include passing the fluid through a particulate matter separation chamber having a seat for a porous filter arrangement, the seat includes structures for aligning the collected particulate matter in a pre-determined spatial arrangement, structures that enhance the fluid flow through the particulate matter separation chamber, and/or structures that promote or retain the porosity and/or compression of the porous filter arrangement housed in the particulate matter separation chamber.

The present invention is also an improved device for collecting a processing a fluid, typically a biological fluid. The device includes a particulate matter separation chamber having one or more of the following: a collection site; a porous filter arrangement including a membrane for separating particulate matter from a fluid and a porous support frit; the porous filter arrangement establishes at least two fluid flow paths through the particulate matter separation chamber; a chamber seat that configures the collected particulate matter in a predetermined spatial arrangement; a particulate matter separation chamber having a concentric channel; a channel having one or more resilient members; a chamber seat having one or more resilient members; a chamber seat or base having posts; a chamber seat having one or more predetermined surface modifications; a chamber seat having one or more elements that promote a predetermined spatial arrangement of particulate matter on the collection site; and structures that enhance the fluid flow through the particulate matter separation chamber.

A device according to the present invention may also include structures that are configured for and/or are adapted to mix the specimen collected in the specimen container. Exemplary structures include, but are not limited to, a specimen container having a cap, or a portion of the cap, that is relatively rotatable; a cap or cap portion that is moveable in relation to the specimen container; and a tube or the like that extends into the specimen container. The tube may include one or more elements for agitating the specimen. The cap may also include a portion that fittingly engages a portion of a cover for particulate matter separation chamber in a liquid tight seal. The cap may also include a portion that fittingly engages a portion of the cover in a liquid-tight but not fluid-tight seal.

A device according to the invention may also include a pump or syringe. The pump or syringe may optionally include one or more elements configured to permit a pre-determined amount of fluid into the pump or syringe.

The present invention also includes preparing a specimen for microscopic examination by processing a fluid using a device according to the invention, and collecting particulate matter on a collection site in the device.

The present invention also includes a method for analyzing matter comprising collecting a fluid in a chamber, collecting particulate matter on a collection site, and transferring the particulate matter collected on the collection site to a microscope slide or the like. Preferably, both collecting steps occur within the chamber.

A device according to the present invention may also include one or more separable elements. In a preferred embodiment of the invention, the device includes a separable particulate matter separation chamber. In a most preferred embodiment of the invention, the device includes a porous filter arrangement at least partially retained in a top portion of the chamber.

The present invention also includes a kit having an assay module that includes a particulate matter collection element according to the invention, a fluid specimen container, and a pump for inducing fluid flow from the specimen container through the assay module.

In a preferred embodiment of the invention, a fluid specimen in a container is in fluid communication with a particulate matter separation chamber or module for separating particulate matter in the fluid and collecting the separated particulate matter in a collection site. In a most preferred embodiment of the invention, the separated particulate matter is collected in a monolayer on the collection site. A preferred embodiment of the invention also includes a hollow tube providing fluid communication between the specimen container and the particulate matter separation chamber. More preferably, the hollow tube includes means for agitating the specimen and/or dispersing the particulate matter in the specimen.

In another embodiment of the invention, the apparatus includes the specimen container and particulate matter separation chamber described above, and a pump, syringe or the like. In this embodiment of the invention, various structures provide a fluid flow path from the specimen container, through the particulate matter separation chamber, and into the pump or syringe.

As used herein, the terms "sample" or "specimen" refer to any fluid in combination with solid matter, such as particulate matter, and from which it may be desirable to collect the particulate component from the sample for the purpose of establishing its identity or presence in the sample. Typically, the fluid component of the sample will be a liquid. However, the fluid may also be air or gas. As an example, it may be desirable to determine the presence of cancer cells or certain proteins in the biological fluid, such as urine. In another example, it may be desirable to evaluate the nature of contaminants, such as molecular contaminants, in ultra-pure water used in the electronics industry. Other exemplary fluids include but are not limited to body fluids, such as blood, spinal fluid, or amniotic fluid; bronchial lavage; sputum; fine needle aspirates; ground water; industrial processing fluids; and electronic or medical dialysis fluids, to identify just a few. It is intended that the type of fluid being processed should not limit the invention.

As used herein, the term "fluid" refers to any fluid for which it may be desirable to collect a component of the fluid for the purpose of establishing its identity or presence in the fluid. Typically, the component in the fluid will be a solid matter, such as particulate matter. For example, the fluid may be air or gas, or a biological fluid, such as urine, and it may be desirable to determine the presence of cancer cells or certain proteins in the biological fluid. In another example, it may be desirable to evaluate the nature of contaminants, such as molecular contaminants, in ultra-pure water used in the electronics industry. Other exemplary fluids include but are not limited to body fluids, such as blood, spinal fluid, or amniotic fluid; bronchial lavage; sputum; fine needle aspirates; ground water; industrial processing fluids; electronic or medical dialysis fluids; to identify just a few. It is intended that the type of fluid being processed should not limit the invention.

As used herein, the term "particulate matter" refers to any substance in a fluid that is capable of collection and evaluation, preferably by cytological examination. Exemplary particulate matter includes, but is not limited to cells or cell fragments, proteins, molecules, polymers, rubbers, stabilizers, antioxidants, accelerators, silicones, alkyds, thiokols, paraffins, thermoplastics, bacteria, pesticides, and herbicides. Specific exemplary polymeric matter include, but is not limited to polyethylene, polypropylene, polyisobutylene, polyacrylonitrile, polyethylene glycol, polyvinylchloride, polystyrene, polysulfide, polymethylmethacrylates, polyethyleneterephthalates, bisphenol A (a common environmental contaminant), ethyl cellulose, nitrocellulose, polyurethane, and nylon. Specific exemplary biological matter includes cancer cells, including distinguishing between metastatic and normal cancer cells; proteins, nucleic acids, antibodies, or the like.

As used herein, the terms "adapted for communication", "communicating," or similar terms refer to any means, structures, or methods for establishing fluid flow through the system, as are well known by practitioners in the art. Exemplary structures are shown in the Figures. For example, a conduit may have a connector adapted to receive or connect to a mated connector on another conduit. As used herein, the term "connector" refers to any structure used to form a joint or to join itself to another piece. These connectors or connections establish a fluid flow path through various elements of the apparatus, assembly, or system. Typical connections include but are not limited to mating connections, such as Luer-type, screw-type, friction-type, or connectors that are bonded together.

As used herein, "adapted for engaging", "engagement", "engaging", or similar terms refers to complementary structures that may align, mesh, mate, or rest near, against, or within each other. Exemplary structures include the connectors described above.

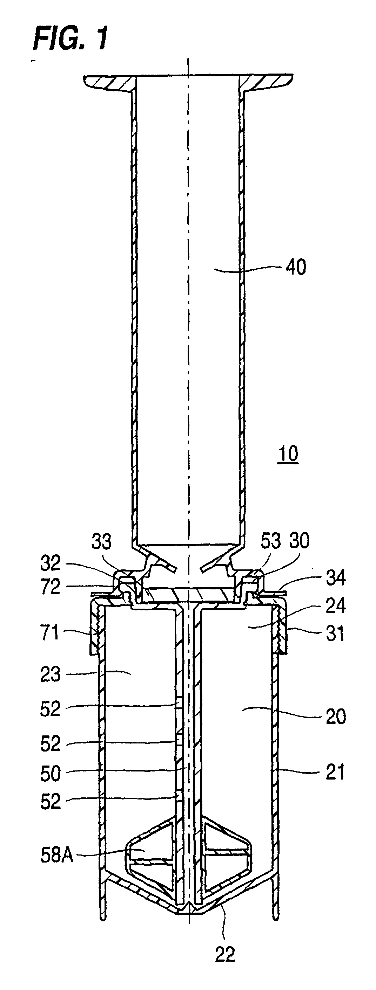

A device 10, according to an exemplary embodiment of the present invention that is shown in Figure 1, includes a specimen container 20 holding a fluid specimen 23, a particulate matter separation chamber 30 having a porous filter arrangement, and a pump 40. Figure 1 also shows a hollow tube 50 that includes a dispersing element 51.

Each of these elements will now be described in more detail.

### THE COLLECTION CONTAINER

In accordance with the invention, specimen container 20 includes any container suitable for holding a fluid 23, preferably a biological fluid. The typical container includes sidewalls 21 and a bottom wall 22 that, in combination, contain the specimen 23. The specimen container 20 also has an open end 24 for collecting, holding, or storing the fluid 23. Typical fluids include, but are not limited to biological fluids, such as body fluids, wastewater fluids, or the like. Typical body fluids include urine or other biological fluids, such as blood, cerebrospinal fluid (CSF), bronchial lavage, sputum or fine needle aspirates.

The configuration and materials used to make the container (and any of the elements that comprise a device according to the invention) can be any of a variety of materials, shapes, and sizes. For example, the cup can be constructed of any material compatible with the fluid to be processed. It will be appreciated that the container and the assembly of the sidewalls to the bottom wall can be any conventional assembly. In a preferred embodiment of the invention, bottom wall 22 is a conical member, as shown in Figure 1. Optionally, bottom wall 22 or sidewall 21 may include one or more fins or the like (not shown) extending into the interior of container 20. Such fins may be desirable an embodiment of the invention described in more detail below in which the sample in the container is agitated by rotation of the container.

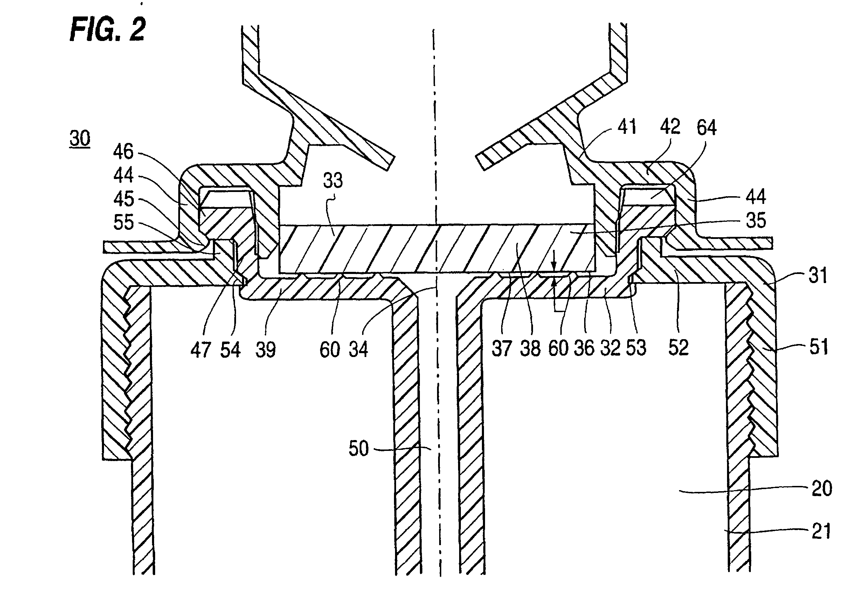

As shown in Figures 1 and 2, a device according to the invention also includes a cap 31. In a preferred embodiment of the invention, the cap 31 is configured or adapted to receive a lower portion 32 of a particulate matter separation chamber 30. The cap 31 may be variously configured to achieve the desired function. A preferred embodiment is shown in Figure 2. The cap 31 may include a downwardly extending member 51 configured to engage sidewall 21 of container 20. It is intended that cap 31 may be any configuration or shape that closes or seals open end 24 of container 20.

The cap also includes portion 52 having an opening 53 adapted to receive the lower portion 32 of the particulate matter separation chamber 30. Although the engagement between cap portion 52 and lower portion 32 may be variously configured, lower portion 32 preferably includes a groove 53 adapted to receive a projection 54 from cap portion 52. In a most preferred embodiment of the invention, the engagement is a snap fit, with the engagement between lower portion 32 and the projection 54 permitting lower portion 32 to rotate relative to cap portion 52. This configuration is preferably liquid tight, and in a most preferred embodiment of the invention, the seal is liquid tight, but not gas (e.g., air) tight.

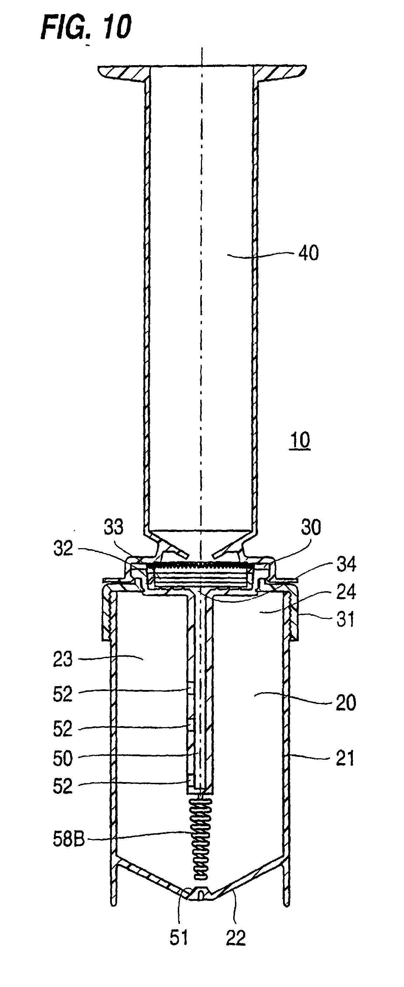

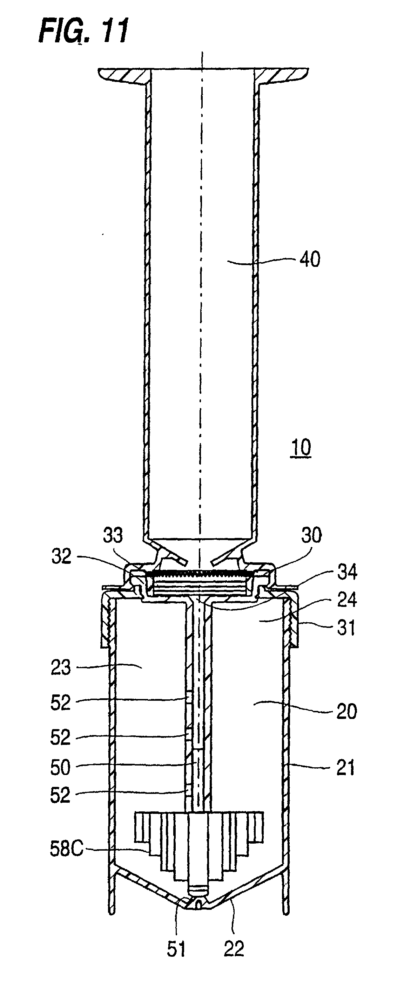

A preferred configuration for the cap 31 will now be described with respect to Figure 1. The cap 31 may be variously configured to achieve the desired function. According to this embodiment of the invention, cap 31 includes structures and means for allowing an outer cap 71 to move in relation to an inner cap 72. Outer cap 71 is preferably fixed to and/or in fluid communication with tube 50. In a preferred embodiment of the invention, the outer cap 71 and tube 50 are relatively rotatable with respect to inner cap 72, when inner cap 72 is tightened on container 23. Such relative motion between outer cap 71 and inner cap 72 moves sample in the container 23 in relation to agitator 58A (Figure 1), brush 58B (Figure 10) or broom 58C (Figure 11).

In the embodiment of the invention that includes the inner and outer caps, it is preferred that the inner and outer caps are adapted to engage each other so that the respective caps do not rotate until the final closing of the cap on the container. It is intended that at least initially, the respective caps act as a unitary cap. When the cap unit is tightened to a pre-determined position, however, it is intended that any structures holding inner cap 72 in place in relation to the outer cap 71 be broken or released so that inner and outer caps rotate freely with respect to one another. For example, inner cap 72 may be used to seal the container and outer cap 71 may snap fit over the inner cap 72. In this embodiment of the invention, a tab or the like on the inside of the outer cap 71 may prevent relative movement between the inner and outer caps when the respective caps are in a first position. Moving the outer cap 71 to a second position, e.g., breaking the tab, permits rotation of the outer cap 71 relative to the inner cap 72. Alternatively, it is envisioned that a temporary spacer (not shown) would initially maintain the inner and outer caps at an axially spaced apart position. After tightening the inner cap 72 on to container 20, the spacer would be removed and the outer cap 71 slid axially over the inner cap 72 to a position that is freely rotatable with respect to the inner cap 72.

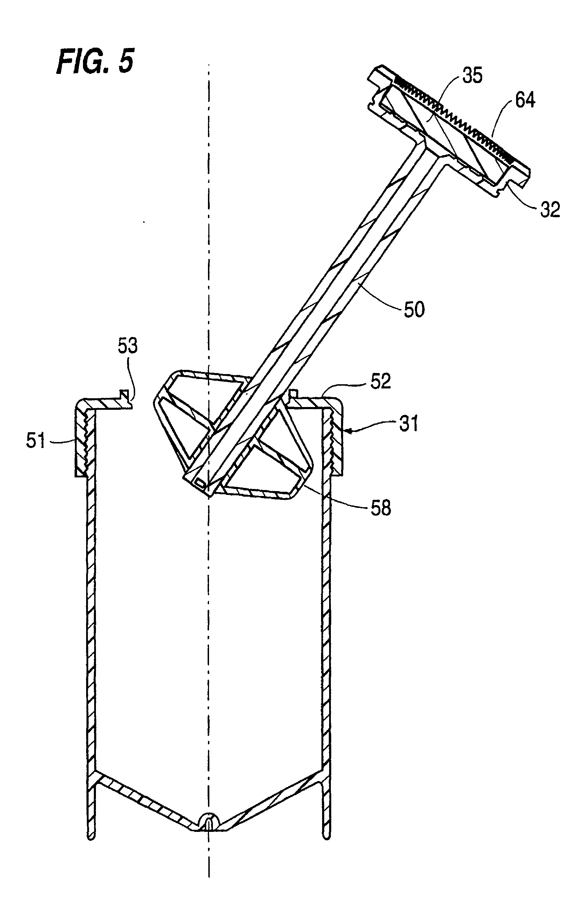

An alternative or additional structure in the embodiment of the invention that includes a cover with a flexible wall 55, preferably circular or elliptical, that engages and or supports a portion 45 of the particulate matter separation chamber 30. In a most preferred embodiment of the invention, the wall 55 includes one or more spaced apart notches (not shown). It is intended that these notches provide a degree of flexibility in the wall so that, if desired, the lower portion of the particulate matter separation chamber 30 can be disengaged from the cap 31 (see, for example, Figure 5).

Figure 5 also illustrates another embodiment of the invention relating to a cap 31 having a slot through which the agitator 58A or broom 58C can be positioned inside the container 20. In a preferred embodiment of the invention, the slot or opening in the cap 31 can be covered with a removable and/or penetrable covering that protects the inside of the container 20 from contamination until the container 20 is ready for use. For example, a brush 58B or the like can be used to collect a cervical sample, the covering can then be removed from the cover 31, and the brush 58B can be placed in the container 20.

According to another preferred arrangement, the inner cap 71 may have a collar (not shown) that coaxially circumscribes tube 50 and extends partially into the container 20. Such a collar redirects the specimen 23 back down into the container 20 during agitation, such as would occur during vortex agitation. This is advantageous insofar as the collar imposes little or no resistance to relative rotation between the inner cap 71 and the outer cap 72. Moreover, it is envisioned that the outer cap 72 may have formed thereon a mating nipple for attaching the tube 50. The mating nipple may be formed on the outer cap 72 so as to extend coaxially within the collar. In this way, a telescoping or breakable tube 50 may be used in its elongated configuration to collect the specimen, and then reconfigured to its collapsed configuration and attached to the mating nipple. Such an arrangement according to this embodiment would further reduce the possibility of sample contamination by minimizing handling of the specimen between the time the sample is collected and the time it is examined.

### PARTICULATE MATTER SEPARATION HOUSING

In accordance with the present invention, a device according to the invention includes a particulate matter separation housing that may be variously configured. An exemplary configuration is shown in Figure 2. Any housing 30 adapted to receive a particulate matter collection assembly 33 may be used.

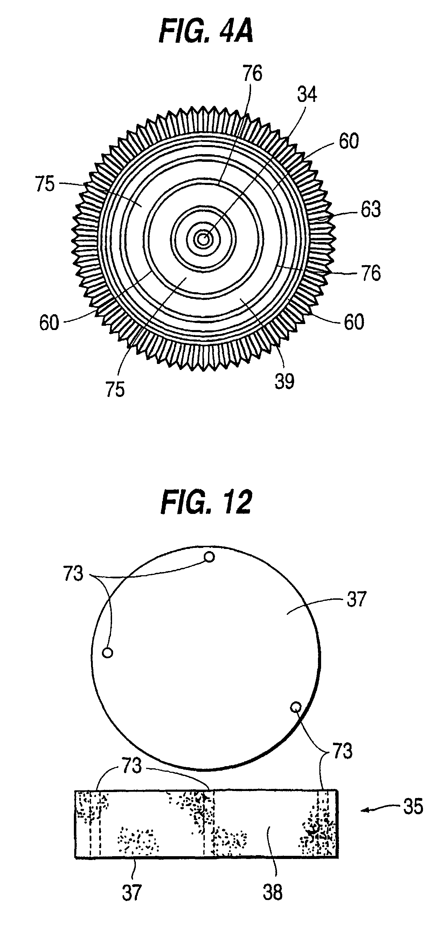

As shown in Figures 1 and 2 the particulate matter separation chamber 30 is preferably a two piece housing formed by a top portion 41 and base portion 32. In a preferred embodiment of the invention, top portion 41 releasably engages base portion 32; however, alternative chamber configurations or assemblies that providing access to the porous filter arrangement 35 are suitable. In a preferred embodiment of the invention, base portion 32 includes a side wall 47, typically circular, that optionally includes a serrated portion 63 (shown in Figure 4A) that engages or communicates with side wall 44 and seat 42 of top portion 41. It has been found that the optional serrated portion 63 of the lower portion 32 facilitates disengaging the lower portion 32 from the top portion 41. Top portion 41 and base portion 32 may be connected or fastened to each other by any mating connection or means that provides a liquid or fluid tight fit, e.g., Luer-type (threaded or not threaded), screw thread-type, friction-type, a tapered mating connection, or snap fit (as illustrated).

Base portion 32 includes a side wall and bottom wall suitable for seating a particulate matter filter assembly 33. Base portion 32 may also include a central bore or aperture 34 communicating with the hollow tube 50. In a preferred embodiment of the invention, hollow tube 50 extends into specimen container 20. In a preferred embodiment of the invention, base portion 32 may be a separate structure that is capable of rotating with respect to the cap 31. In order to achieve ease of centrifugal rotation while maintaining a liquid-tight assembly, base portion 32 may matingly engage base 31 through a tongue and groove arrangement (see Figure 2).

In accordance with an embodiment of the invention, base portion 32 of the particulate matter separation chamber 30 housing includes a bottom wall or seat 39. As shown in Figures 4A-4C, seat 39 may include one or more spaced apart ribs or projections 60. Projections 60 are preferably of a configuration, size, and shape sufficient to prevent porous arrangement 35 flush contact with seat 39. In the embodiment shown in Figure 4A, projections 60 are concentric rings.

Alternative configurations are described in more detail below. In a preferred embodiment of the invention, projections 60 function in one or more of the following ways: projections 60 may break the surface tension between porous filter arrangement 35 and seat 39 during use; when porous filter arrangement 35 is to be pulled away from seat 39, first porous medium 36 does not remain in contact with seat 39; projections 60 may evenly distribute pressure of the porous filter arrangement in the particulate matter separation chamber 30; projections 60 may prevent or suppress compression of the porous filter arrangement; and projections 60 may be configured to distribute any collected particulate matter in a pre-determined configuration or spatial distribution.

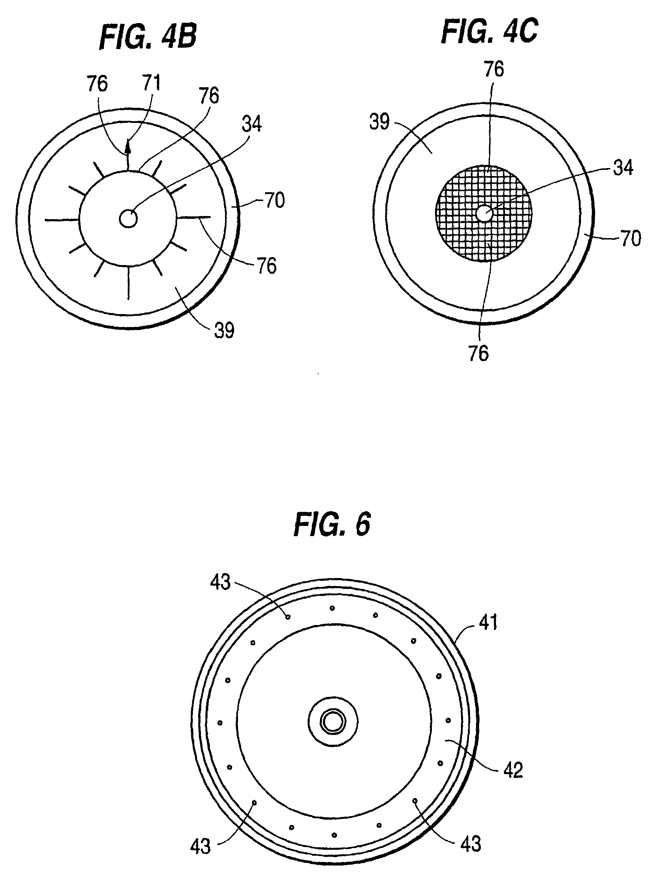

In accordance with the present invention, the surface of seat 39 may include one or more structures, configurations, or surface textures that promote the ability of the porous filter arrangement 35 to release from the seat 39, that promote a pre-determined spatial distribution of particulate matter on the collection site, and/or prevent or suppress compression of the porous filter arrangement 35. One embodiment of the invention includes concentric projections, such as projections 60 described above. Other configurations include, but are not limited to a grid, cross-hatching or the like, concentric squares or rectangles, or a series of continuous or separated structures, nubs, protuberances, granulations, or the like (see Figures 4B and 4C). It is intended that any element, structure, or chemistry that provides a texture to the surface of the seat 39 for accomplishing the above stated functions is suitable for use with the present invention.

In a preferred embodiment of the invention, the surface of the seat is configured into cross-hatching (see Figure 4C). In another preferred embodiment of the invention, the surface of the seat is configured into a sundial or clock face structure (see Figure 4B). Both of these embodiments, as well as other surface configurations disclosed herein, promote the collection of particulate matter on the collection site in a pre-determined spatial arrangement. The configurations shown in Figures 4B and 4C are particularly desirable because the imprint of the surface treatment of the seat may be transferred to the microscope slide and used to locate and identify specific particulate matter, such as a cancer cell, using a coordinate system. It has been found that a greater portion of particulate matter collects in regions on the collection site corresponding to or opposite areas 75 of the seat. Conversely, high spots 76 are regions that correspond to areas of where smaller amounts of particulate matter collects on the collection surface. These regions are imprinted on the microscope slide when the collection surface is placed in contact with the slide.

For example, a technician reading a microscope slide according to the present invention may be able to identify and locate a cell of interest by noting that the particular cell can be found at an angular position corresponding to 2 O'clock on the clock face configuration shown in Figure 4B. Imprinting a microscope slide in such a manner significantly speeds reviewing slides and significantly improves the ability of a technician to find previously identified matter of interest. Included with the invention are one or more structures on the seat surface that provide positive orientation of the particulate matter as it is collected on the collection site and transferred to the microscope slide. For example, a suitable coordinate-identifying structure may be an arrow 71 or the like, as shown in Figure 4B.

In accordance with another embodiment of the invention, the seat 39 and/or lower portion 32 may optionally include a channel 70 or the like, examples of which are shown in Figures 4B, 4C and 7-9. In a preferred embodiment of the invention, seat 39 slopes slightly outward toward the channel 70. The slight slope of the seat 39 and the channel 70 promote enhanced fluid flow through the particulate matter separation chamber 30 and decreases the surface tension of the seat 39 on the filter arrangement 35, both of which promote the capability of the porous filter arrangement 35 to disengage from the lower portion 32 of the particulate matter separation chamber 30. This aspect of the invention is another structure(s) that promote release of the porous arrangement.

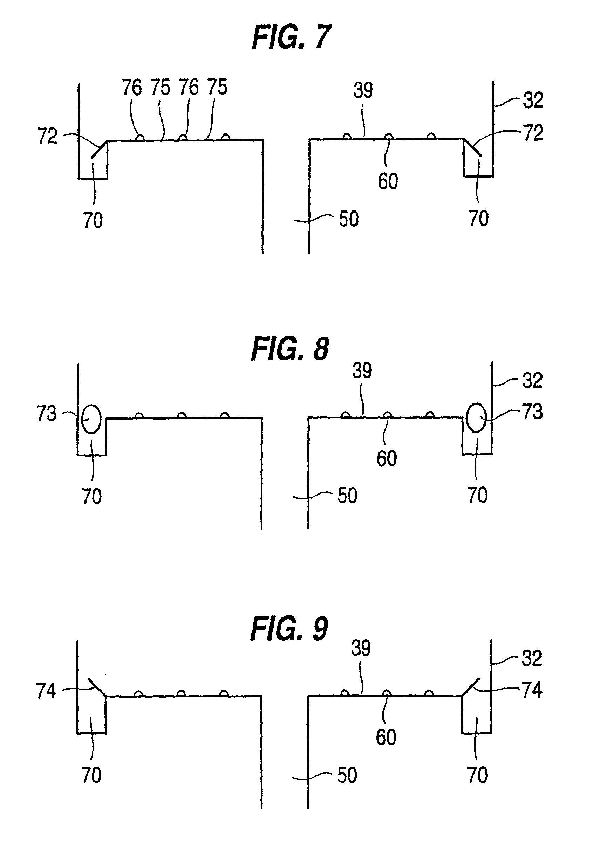

Additional structures are shown in Figures 7-9 that address or are involved with promoting fluid flow through the particulate matter separation chamber 30 and also are involved in the release of the porous filter arrangement 35 from the lower portion 32. Figure 7 shows flap 72 that extends downwardly into channel 70 from the lip of seat 39. Figure 8 shows an O-ring 73 or the like that is positioned in the channel 70, preferably so that a top surface of the O-ring 73 is slightly above the plane of seat 39. This insures that O-ring 73 will engage a portion of porous filter arrangement 35 when positioned in the lower portion 32. Figure 9 shows a flap 74 that extends upwardly from an outer portion of seat 39, insuring that flap 74 will engage a portion of porous filter arrangement 35 when positioned in the lower portion 32. In a preferred embodiment of the invention, flap 72, O-ring 73, and flap 74 are made of a resilient material. The preferred configuration is that shown in Figure 9.

In accordance with the invention, the particulate matter separation chamber 30 is configured to receive a porous arrangement 35 having a particulate matter collection site 36 adapted to collect particulate matter as fluid containing the particulate matter passes through the chamber 30.

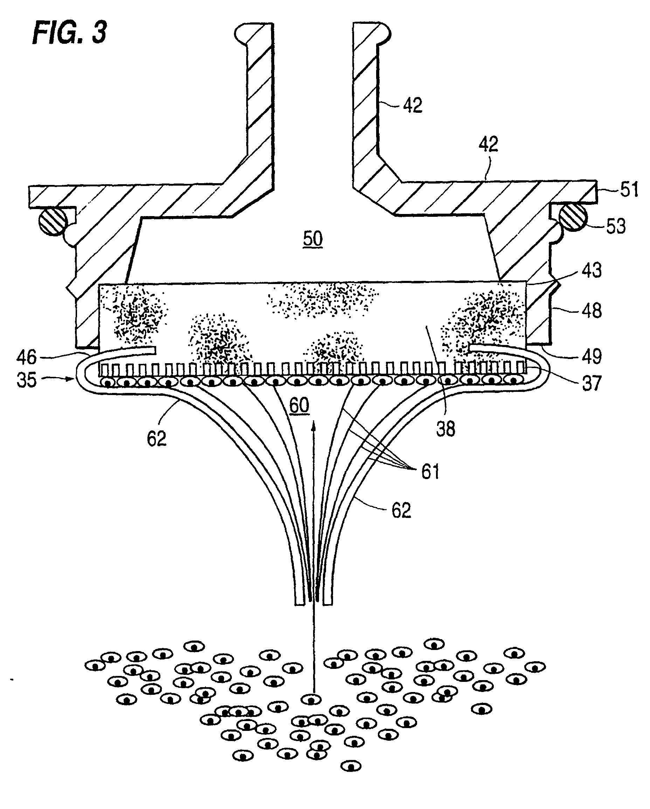

Porous arrangement 35 having a collection site 36 adapted to collect matter may be positioned across a fluid flow path, the collection site 36 communicating with hollow tube 50. The porous arrangement 35 within the matter separation chamber is preferably adapted to define at least one fluid flow path having first and second branches, the first branch 61 extending through the collection site 36 and the second branch 62 bypassing the collection site 36 (e.g., see Figure 3).

In a preferred embodiment, the invention includes a porous filter arrangement 35 having a first porous medium 37, suitable for preventing the passage of particulate matter therethrough, and a second porous medium 38, suitable for allowing fluid to pass therethrough. The second porous medium 38 may or may not be capable of removing particulate matter from the fluid 23, a design choice according to the needs of a particular device. In a preferred embodiment, the first porous medium 37 is suitable for capturing or collecting particulate matter, and even more preferably, capturing or collecting particulate matter in a uniform or single layer. A preferred embodiment also includes a second porous medium 38 that is suitable as a support for the first porous medium 37.

The nature of the material used to make the porous media, the compatibility of the materials chosen for the porous media with one another and with the liquid to be processed are all factors to be considered in selecting a particular material for a porous medium for a given application.

Porous filter arrangement 35 may include a unitary structure having a first porous medium 37 of density and/or pore size suitable to prevent the passage of cells therethrough and a second porous medium 38 of density and/or pore size suitable for passing the fluid therethrough.

In a preferred embodiment, the porous filter arrangement 35 includes a first porous medium 37 comprising a porous polycarbonate membrane, suitable for preventing the passage of particulate matter therethrough. The porous filter arrangement 37 may further include second porous medium 38 comprising a depth filter or frit. The depth filter may be made of polypropylene or high-density polyethylene POREX® porous plastics. In a preferred embodiment of the invention, the second porous medium 38 may include a serrated or saw-tooth downstream portion 64, an example of which is illustrated in Figure 2. It is intended that portion 64 is a structure and configuration that reduces or ameliorates compression of the porous filter arrangement 35 when it is positioned in the particulate matter separation housing 30.

It should be noted that various types of porous filter arrangements 35 could be used interchangeably with that of the present embodiment. While a polycarbonate membrane 37 is especially suitable for use in the cytology collection apparatus of the present invention, other porous membranes are also suitable. Exemplary porous membranes are well known in the art, and are disclosed in U.S. Patents 5,471,994 and 5,301,685, which correspond to World Patent Number 94/03103.

The porous membrane 37 preferably has a pore size from about 0.22 microns to about 8 microns, more preferably from about 1 micron to about 6 microns, most preferably about 2 microns, which allows it to trap particulate matter, e.g., cells, which are more than 3 microns in size. The membrane is suitable to allow fluid flow to pass therethrough while preventing the passage of particulate matter. The second porous medium 38 is suitable for passing fluid therethrough and may also be capable of removing particulate matter from the fluid 23. The pore size of the second porous medium 38 may range from about 5 microns to about 60 microns, preferably from about 15 microns to about 45 microns, most preferably about 35 microns.

As one skilled in the art will recognize, adjusting the pore size of the porous membrane 37 and the porous depth filter 38 in accordance with the type and/or size of matter to be collected permits the collection of the particulate matter on the collection site. In a preferred embodiment of the invention, the pore size is chosen so that a uniform layer of matter, preferably a monolayer of matter, is formed on the collection site. For example, from about 3 µm to about 40 µm or more has been shown to be effective, but it is intended that the invention should not be limited to a certain range of pore size.

In a most preferred embodiment of the invention, first porous medium 37 is attached to second porous medium 38 using an adhesive that is soluble in liquid. Such soluble adhesives include but are not limited to sugar compositions, gels, and the like.

The first porous medium 37 and the second porous medium 38 may be positioned in any fashion that functions as described herein. As one skilled in the art will recognize, the porous filter arrangement 35 may be variously configured and positioned as needed to achieve a particular result. For example, the first and second porous media may be separate, spaced apart media; the two media can be laminated together; the first medium can be integral with or removably engaged with the second porous medium; or the collection element may comprise a zone of higher density which mimics the function of the first porous medium as described above, and zone of lower density which mimics the function of the second porous medium as described above. Choice of these various configurations are well within the skill of practitioners in the art. Variations on the structure and composition of the porous arrangement will be described in more detail below.

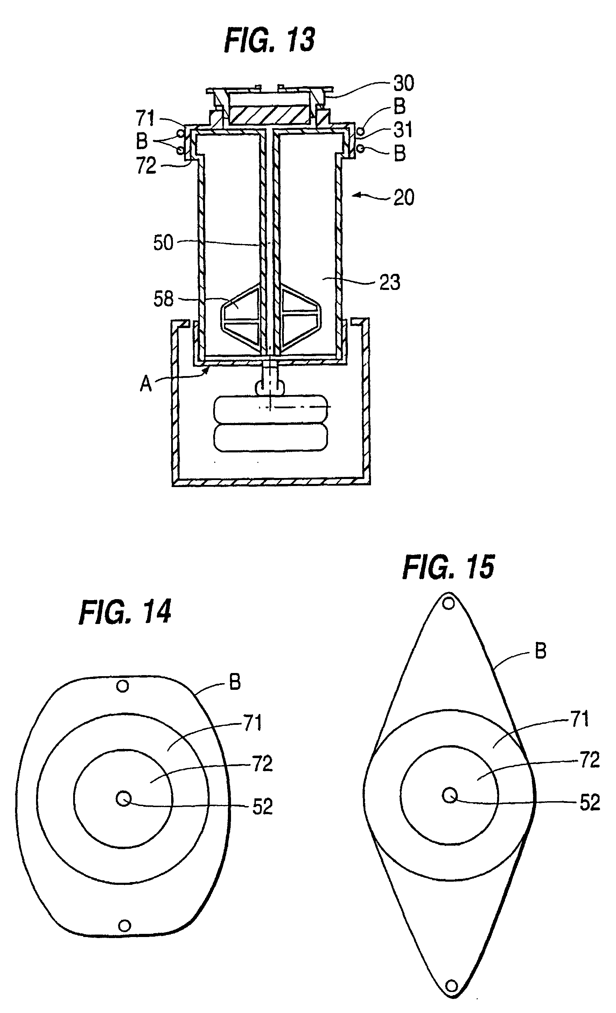

As shown in Figure 12, a porous support 38 with at least one through bore 73, preferably a bore positioned near the circumference of the porous support 38, provides a direct conduit for suction so that a filter membrane 37 is retained on the porous support 38 when the particulate matter separation chamber 30 is opened to expose the membrane 37 for further processing.

In another embodiment of the invention, lower portion 32, tube 50, and fins 58 form an integral unit, and may be separated from cap 31 to facilitate removal of the integral structure from container 20. An exemplary structure of this embodiment of the invention is shown in Figure 5.

### PUMP

In accordance with the invention, specimen container 10 includes a pump 40. In a preferred embodiment of the invention, pump 40 is a syringe or the like for altering differential pressure within the apparatus so that fluid can be drawn from the specimen container 20 through the particulate matter separation chamber 30.

In accordance with the present invention, the pump 40 may be variously configured. In a preferred embodiment of the invention, pump 40 includes an end that forms the cover portion 41 of the particulate matter separation chamber 30. Cover portion 41 includes a seat 42 or the like configured to engage a downstream portion of porous filter arrangement 35. In a preferred embodiment of the invention, the seat 42 positions porous filter arrangement 35 in the cover so that porous filter arrangement 35 does not move during use. In a most preferred embodiment of the invention, seat 42 includes a plurality of projections or posts 43 of a size, shape, and number to position the porous filter arrangement 35 in the particulate matter separation chamber 30, to promote substantially even distribution of pressure against the porous filter arrangement 35, and to reduce or prevent compression of the porous filter arrangement 35 that could interfere with fluid flow through the porous filter arrangement 35.

In a preferred embodiment of the invention, cover portion 41 removably engages bottom portion 32 to form the particulate matter separation chamber 30. Cover portion 41 may engage bottom portion 32 in any manner and with any structures that allow cover portion 41 to disengage bottom portion 32. In a preferred embodiment of the invention, illustrated in Figure 2, cover portion 41 includes a downwardly extending side wall 44 having a flange 45 or the like adapted to releasably and/or resiliently engage a shoulder 46 or the like on bottom portion 32.

Movement of fluid through the collection apparatus may be effected by maintaining a pressure differential between a source of fluid and the destination of the fluid. Exemplary means of establishing this pressure differential may be by applying pressure to any part of the system on the inlet side of the particulate matter separation chamber 30 (e.g., the specimen container 20); applying a vacuum to any part of the system on the outlet side of the housing (e.g., the syringe 40); or any form of pump, such as an autovial spunglass filter (manufactured by Genex Corporation); gravity head; or a flexible, collapsible container, such as a specimen container, which may be squeezed to force fluid through the matter collection apparatus and into the syringe. In a preferred embodiment of the invention, a syringe draws fluid from a collection cup through the housing.

### HOLLOW TUBE

In accordance with a preferred embodiment of the present invention, specimen container 20 includes a tube 50 or the like for drawing fluid 23 into the particulate matter separation chamber 30. Typically, tube 50 will be hollow and open or openable at both ends. Tube 50 includes open end 51 near the bottom of the collection chamber 23, and may include one or more apertures 52 into tube 50. Open end 51 and/or apertures 52 permit different fluid layers as well as sediments to be simultaneously tested when the fluid is drawn into the particulate matter separation chamber 30.

In accordance with another embodiment of the improved invention, hollow tube 50 includes at least one projection or fin 58A or the like, as shown in Figure 1. In a preferred embodiment of the invention, hollow tube 50 is rotatable and fin 58A stirs the liquid specimen, and in a most preferred embodiment, disperse cells and/or particulate matter, and/or to disrupt any large particulate matter such as mucoid bodies. In another preferred embodiment of the invention, hollow tube 50 and lower portion 32 are of unitary construction, and the lower portion 32, tube 50, and fin 58A are movable in relation to the specimen container 20. For example, if the container is rotated, optional fins in the side and/or bottom walls of the container may create concentric movement of the sample in the container, movement that will be disrupted by the presence of fin 58A. Alternatively, lower portion 32, tube 50, and fin 58A may be rotated within a stationary container.

As shown in Figures 10 and 11, as an alternative embodiment of the invention, agitator 58 may comprise fibers, a brush, swab, or broom or the like. Preferably, such fibers or brush are suitable for dispersing particulate matter in the container when the sample is vortexed in relation to the agitator, brush, or broom. In a most preferred embodiment of the invention, the brush or broom is also suitable for use in collecting particulate matter from a patient, e.g., a cervical brush or broom or the like. It is intended that the brush can be fixed to a portion of the cap 31, or the cap 31 may include a slot, collar or the like for matingly engaging a portion of the handle at the opposite end of the brush.

### MIXER

Figures 13-15 show an apparatus for a semi-automated method according to a preferred embodiment of the invention. In particular, Figures 13-15 show a most preferred embodiment comprising a support sleeve A for positioning and rotating the container and the inner cap 72. In the most preferred embodiment of the invention, the outer cap 71 is engaged by one or more resilient bands B that in a loosened or first position (Figure 14) do not engage outer cap 71, and in a tightened or second position (Figure 15) engage and hold the outer cap 71 while the inner cap 72 and container 20 are rotating. In an alternative embodiment, belt B may be a drive belt that rotates the outer cap 71, tube 50 and agitator 58, as a unit, with respect to container 20 and inner cap 72.

### KIT

The present invention is also directed to a particulate matter collection and testing kit containing the collection apparatus 10 as an integral unit. The kit may include at least one specimen container 20, at least one particulate matter separation chamber 30, at least one pump 40, and at least one porous filter arrangement 35. A kit according to the invention may also include replacement filters, replacement disposables, and/or other components or solutions typically used during particulate matter testing or examination procedures, e.g., cytological examinations.

### FIXATIVE

A composition according to the invention includes one or more solvents, preferably an alkanol, between about 35% and about 45% by volume; ketone, between about 2 % and about 3 % by volume; a diluent, preferably a diol or triol, from about 1 % to about 3 % by volume; a crosslinker, preferably an aldehyde, from about 0.4% to about 3% by volume; glycerol, from about 0.5 % to about 2 % by volume; one or more detergents and/or dispersing agents, preferably non-ionic, from about 0.01 % to about 0.05% by volume; and a buffer, from about 45 to about 65 % by volume. In a preferred embodiment of the invention, the pH of the composition is between about 4 and about 7.

The present invention also includes a method of preparing particulate matter, such as cells and the like, for cytological or histological examination comprising collecting particulate matter in a uniform layer, preferably a monolayer, and fixing the cells in a composition according to the present invention.

Table 1 summarizes the range and preferred concentrations of the components of a fixative formulation according to the present invention.

**TABLE 1**

| Component | range (by volume, %) | preferred (by volume. %) | example |
|---|---|---|---|
| solvent | 35 -- 45 | 37 -- 42 | alkanol |
| ketone | 2 -- 3 | 2.1 -- 2.4 | acetone |
| diluent | 1 -- 3 | 1.6 -- 1.9 | diol, triol |
| glycerol | 0.5 -- 2 | 0.8 -- 1.2 | glycerol |
| crosslinker | 0.4 - 3 | 0.6 - 0.8 | aldehyde |
| detergent | 0.01 - 0.05 | 0.02 | Nonidet P40 |
| buffer | 45 - 65 | 50 - 55 | Tris |

A composition according to the invention includes one or more solvents to penetrate the tissue or cells, dehydrate the cells, and/or inhibit bacterial and vital activity. In a preferred embodiment of the invention, the solvent is a mixture of alkanols, which penetrate slowly, and when combined with other reagents, fixes the sample rapidly. It denatures protein by precipitation, precipitates glycogen, and dissolves fats and lipids. The alkanol can be any of thee well known alcohols having one to four carbons, e.g., methanol, ethanol, n-propanol, isopropanol, n-butanol, and various branched butanols. The most preferred solvent is a mixture of methanol and isopropanol, typically about 30% and about 10% by volume, respectively.

The ketone is a fixative with similar action to that of alcohol, except that glycogen is not well preserved. The ketone acts as a fixative and additionally enables the overall composition to penetrate the cells. The preferred ketone is acetone.

The diluent forms a coating over the specimen and helps protect from the effects of drying. The preferred diluent is a diol or a triol, most preferably polyethylene glycol (PEG), e.g., PEG-1500 (average molecular weight of about 1450) also called Carbowax.

Glycerol prevents the drying of cells during sample processing. Cells that have been kept in fixative solution for an extended time typically become rigid due to the fixing process and are less able to spread on the slide. Glycerol helps the cells to flatten on the slide.

The crosslinker reacts with protein end-groups to crosslink molecules and produces an insoluble product. Protein groups involved include amino, imino, and amido, peptide, hydroxyl, carboxyl and sulfhydryl. Methylene bridges are also commonly formed between similar groups such as NH2 and NH but are thought to be reversible by washing in water. Some crosslinkers such as formaldehyde are an antiseptic.

The preferred crosslinkers are aldehydes, most preferably formaldehyde.

The detergent is non-ionic detergents and dispersing agents used for solubilization of proteins and membrane components to diminish cellular aggregation. The preferred detergents are Nonidet P40 or Triton X-100, both well-known detergents.

The buffer maintains the solution at a pH between about 4 and about 7, and provides a medium for transportation. The preferred buffer is Tris, a well-known buffer. In accordance with the present invention, the buffer may also include a fixative that precipitates nucleoproteins and one or more osmolarity maintainers. The preferred nucleoprotein precipitator is acetic acid glacial, typically in a range from about 0.2 % to about 0.3% by weight, and helps maintain the buffer between about 7.4 and about 7.8 pH. The preferred osmolarity maintainers are dextrose, typically in a range from about 0.1% to about 0.2% by weight, and sodium chloride, typically in a range from about 0.7% to about 0.8 % by weight.

In the preferred embodiment, the active fixative ingredients described may be dissolved in a suitable solvent such as distilled water, and this solution can then be used as a fixative agent in a number of ways as would be obvious to one skilled in the art. For example, the fixative solution can be used to preserve samples of tissue that are being shipped or carried to an examination site. In this process, small vials or jars that have liquid tight seals are filled with the reagent of the invention, and tissue samples are placed in the reagent-containing vial to preserve the samples until they reach an area where further processing can occur. Any suitable diluent that does not change the important chemical and physical characteristics of the formulation may be used.

Tissues prepared for study using the fixative of the invention can be prepared for histological study in any known conventional manner, such as through the use of paraffin, sectioning equipment, staining, mounting on slides, or other common steps utilized prior to microscopic or other examination. The present invention thus provides a safe, convenient and effective fixative solution that can be utilized in the many known histological procedures that employ such solutions.

### METHOD

The present invention also includes a method for removing particulate matter from a fluid, and for transferring particulate matter, such as cells, to a microscope slide. In contrast to currently available methods, the use of membrane filtration provides a method of depositing cells evenly over a microscope slide with minimal overlap. This allows for clear observation and optimal diagnostic accuracy.

A method includes collecting a fluid sample containing particulate matter in a collection container 20. The container 20 is then capped with an assembly that includes one or more of the following: cap 31, particulate matter separation chamber 30, and pump 40. Pump 40 is then activated to pull fluid from container 20 through particulate matter separation chamber 30 into pump 40, e.g., by withdrawing the piston in a syringe.

When the fluid is pulled from the container 20 to the pump 40, fluid will flow through porous filter arrangement 35 as shown in Figure 3, so that a monolayer of particulate matter is formed on collection site 37. Once the monolayer of cells is formed, fluid flow is reduced in the center of porous filter arrangement 35 and increases towards the edges of the porous filter arrangement 35. This may be due to the blockage of fluid flow by the collected cells as they form the monolayer on the surface of the porous filter arrangement 35. When the monolayer has mostly covered the surface 45 of the porous arrangement, the flow of fluid bypasses the first porous medium 37 and passes through the extended side area of the second porous medium 38. Thus, the area of the second porous medium 38 extending beyond an end wall or skirt of the top portion acts as a vent (with low resistance to flow) that prevents cells piling up or collecting in more than a monolayer. Fluid may be passed back and forth through the porous arrangement as many times as desirable.

Pump 40 may then be disconnected from base 31, and thereby exposing porous filter arrangement 35. Once porous filter arrangement 35 is removed from lower portion 32, easy access is gained to first porous medium 37. Alternatively, disengaging top portion 41 of pump 40 from lower portion 32 may also remove porous arrangement 35 from well 32.

The first porous medium 37 may then be pressed against a microscope slide to allowing particulate matter collected on the collection site to be transferred, as they were collected, onto the slide. This allows a cytological examination to be performed on the cells by the practitioner without the interference of the pores in the membrane or delay due to processing requirements.

Since cellular detail is dependent on fixation, it is preferred that cells be fixed immediately after being deposited on the slide. Too long a delay between preparation and fixation may expose the cells to drying, which may be detrimental to the cellular structure. Moreover, air-drying artifacts can adversely affect the subsequent staining results. An exception is when the cells are stained with Wright-Giemsa, where air-drying is used as the fixation step.

In an another embodiment of the present invention, the monolayer of cells may be fixed directly on the collection site. This may be carried out by first depositing a monolayer of cells on the collection site of the cytology collection apparatus as described above and subsequently passing a solution containing a fixative, such as alcohol or acetone, through the cytology collection apparatus. Of course, in the most preferred embodiment of the present invention, the above-described fixative would be used.

### ALTERNATIVE CONFIGURATIONS

The matter collection apparatus or module described above may be used in combination with other suitable filtration or treatment devices. Exemplary devices include other debris and/or assay devices or modules that may be attached to housing 10. Typically, these additional modules will include a housing having an inlet and an outlet, and will include a filtration, assay, or detection element positioned across the fluid flow path in the housing. For example, the apparatus may comprise a housing including inlet and outlet ports defining a flow path between the inlet and the outlet; a filter positioned across the flow path; and a freely movable chromatography/assay element, such as substrate beads, positioned on the outlet side of the filter. The chromatography/assay element can freely mix with the matter in the fluid, capture the matter, and can then be assayed for the presence of the matter. Suitable devices include those disclosed in U.S. Patents 4,953,561; 5,224,489; 5,016,644; 5,139,031; 5,301,685; 5,042,502 and 5,137,031.

Included within the scope of the present invention is producing a single slide from a patient sample, producing multiple slides from a single patient sample, or producing multiple slides from multiple patient samples. It is intended that a patient sample may be processed in a single shot, batch, or continuous manner. Additional slides for other stain applications can be easily prepared. Human papilloma virus testing, for example, by newer methods such as immunocytochemistry or in-situ hybridization can be performed on the additional slides. As oncogene products or other immunocytochemical tests are developed, more slides may be necessary. The different fixations that these tests may need can easily be incorporated into the procedure since the preparation does not require the slides to be fixed in only one way.

The most widely used stain for visualization of cellular changes in cytology is the Papanicolaou staining procedure. This stain, which is used for both gynecologic and non-gynecologic applications, is basically composed of blue nuclear and orange, red and green cytoplasmic counterstains. The nuclear stain demonstrates the chromatic patterns associated with normal and abnormal cells, while the cytoplasmic stains help to indicate cell origin. The success of this procedure can be attributed to the ability to observe a number of factors, including definition of nuclear detail and cell differentiation. This staining procedure also results in a multicolor preparation that is very pleasing to the eye, possibly reducing eye strain This same slide preparation procedure can be used for virtually all forms of cytology.

Furthermore, the use of completely contained disposable components addresses biohazard concerns. Ultimately, the enhanced presentation of cells, yielding improved cytologic interpretation, may expand the role of cytology by providing more consistent and reliable patient diagnosis.

Also, captured microorganisms can be cultured in culture medium. After a monolayer of cells has been collected in the cytology collection apparatus, fluid may be used to back-flush the collection site, thereby transferring any collected microorganisms from the collection site.

In bacteria testing, the first porous medium can be used for culturing with a Qualture device (not shown) to determine the presence of specific bacteria colonies. The Qualture device is a plastic capsule containing a filter membrane and four nutrient pads of dehydrated, selective media.

The Qualture technique is more sensitive than the agar plate method and more rapid in determining a presumptive diagnosis. The device screens, isolates and presumptively diagnoses bacterial isolates in one step most often in 4-6 hours. Tests have demonstrated that recovery from fifty milliliters of fluid is excellent and sensitive.

## Claims

1. An apparatus (10) for processing a liquid containing particulate matter, comprising:
a container (20) including a cap (31) fixedly engaging said container;
a porous arrangement (33) in a housing (30), said housing comprising a first portion (32) relatively rotatably engaging said cap; and
a pump (40) engaging a second portion (41) of said housing;
said housing separating between said first portion and said second portion, and said second portion retaining said porous arrangement.

2. The apparatus (10) of claim 1, wherein said first portion (32) engages said cap with a liquid tight seal.

3. The apparatus (10) of claim 2, wherein said liquid tight seal is not fluid tight.

4. An apparatus (10) for separating particulate matter from a liquid comprising:
a container (20) including a cap (31), said cap comprising a first portion (72) fixedly engaging said container and a second portion (71) relatively rotatable with respect to the first portion;
a porous arrangement (35) in fluid communication with the container and suitable for removing particulate matter from a liquid, said porous arrangement being positioned in a top portion (32) releasably engaging said second portion, said top and second portions defining a housing (30); and
a pump (40) in fluid communication with said housing.

5. The apparatus (10) of claim 1 or 4, wherein said cap (31) comprises a tube (50) that extends into said container (20).

6. The apparatus (10) of claim 5, wherein said tube (50) comprises at least one agitating fin (58A) on the end of the tube that extends into said container (20).

7. The apparatus (10) of claim 1 or 4, wherein said pump (40) comprises one or more stops for drawing a pre-determined amount of liquid into the pump.

8. An apparatus (10) for processing a liquid containing particulate matter, comprising:
a porous arrangement (35) suitable for removing particulate material from a liquid containing particles; and
a housing (30) supporting said porous arrangement in an interior chamber, the housing including a first portion (32) releasably receiving said porous arrangement and a second portion (41) retaining said porous arrangement, said housing separating between said first and second portions, said first portion having a seat (39) confronting said porous arrangement, said seat defining a predetermined hydraulic relationship between said housing and said porous arrangement.

9. The apparatus (10) of claim 8, wherein said second portion (41) comprises at least one nub engaging the porous arrangement.

10. The apparatus (10) of claim 8, wherein said first portion (32) comprises a serrated portion (63) for disengaging the second portion.

11. The apparatus (10) of claim 8, wherein said predetermined hydraulic relationship is defined by one or more structures configured to reduce surface tension of said lower portion with respect to said porous arrangement.

12. The apparatus (10) of claim 8, wherein said first portion (32) comprises one or more structures (60) configured to reduce the capability of retaining the porous arrangement in said first portion.

13. The apparatus (10) of claim 8, wherein said second portion (41) comprises one or more structures configured to increase the capability of retaining the porous arrangement in said second portion.

14. The apparatus (10) of claim 8, further comprising a channel (70) around said seat.

15. The apparatus (10) of claim 14, further comprising a resilient member positioned in said channel.

16. The apparatus (10) of claim 15, wherein said resilient member is an O-ring (73) or a flap (72)(74).

17. The apparatus (10) of claim 8, wherein said seat (39) comprises a pre-determined surface texture.

18. The apparatus (10) of claim 17, wherein said seat (39) comprises a slope extending radially from a central core.

19. The apparatus (10) of claim 17, further wherein said seat (39) further comprises a flap engaging the porous arrangement.

20. The apparatus (10) of claim 17, wherein the surface texture comprises projections (76) in a pre-determined pattern.

21. The apparatus (10) of claim 20, wherein the pattern is a grid.

22. The apparatus (10) of claim 20, wherein the pattern is a clock face.

23. An apparatus (10) for processing a liquid containing particulate matter comprising:
a container (20) including a cap (31) fixedly engaging said container;
a porous arrangement (33) in a housing (30), said housing comprising a first portion (32) engaging said cap, said first portion having a tube (50) that extends into said container, said first portion relatively rotatably engaging said cap, said first portion releasably receiving said porous arrangement; and
a pump (40), said pump comprising a portion adapted to engage a second portion (41) of said housing;
said housing being adapted to separate between said first portion and said second portion, and said second portion being adapted to retain said porous arrangement.

24. The apparatus (10) according to any one of claims 1-23, further comprising:
a motor agitating the liquid by relative rotation between said container and said housing.

25. A method for separating particulate matter from a liquid, comprising:
passing liquid containing particles through a porous arrangement suitable for removing particulate matter from said liquid, said liquid passing from within a container, said porous arrangement being received in a first portion of a housing, said first portion relatively rotating with respect to said container and extending into said container for fluid communication with the liquid; and
separating a second portion of said housing from said first portion, wherein said separating step includes retaining said porous arrangement in said second portion of said housing.

26. The method according to claim 25, further comprising:
agitating the liquid in said container with a fin fixed to said first portion extending into said container.

## Patentansprüche

1. Vorrichtung (10) zur Verarbeitung einer Feststoffe enthaltenden Flüssigkeit, mit:
- einem Behälter (20) einschließlich eines fest mit dem Behälter in Eingriff stehenden Deckels (31);
- einer porösen Anordnung (33) in einem Gehäuse (30), wobei das Gehäuse einen mit dem Deckel relativ drehbar in Eingriff stehenden ersten Abschnitt (32) und
- eine mit einem zweiten Abschnitt (41) des Gehäuses in Eingriff stehende Pumpe (40) aufweist;
wobei das Gehäuse eine Trennung zwischen dem ersten und zweiten Abschnitt bildet und der zweite Abschnitt die poröse Anordnung zurückhält.

2. Vorrichtung (10) nach Anspruch 1, bei der der erste Abschnitt (32) mit dem Deckel über eine flüssigkeitsdichte Dichtung in Eingriff steht.

3. Vorrichtung (10) nach Anspruch 2, bei der die flüssigkeitsdichte Dichtung nicht fluiddicht ist.

4. Vorrichtung (10) zur Trennung von Feststoff von einer Flüssigkeit, mit:
- einem Behälter (20) einschließlich einem Deckel (31), wobei der Deckel einen fest mit dem Behälter in Eingriff stehenden ersten Abschnitt (72) und einen relativ zum ersten Abschnitt drehbaren zweiten Abschnitt (71) aufweist;
- einer porösen Anordnung (35) in Fluidverbindung mit dem Behälter und geeignet zum Entfernen von Feststoffen aus einer Flüssigkeit, wobei die poröse Anordnung in einem lösbar mit dem zweiten Abschnitt in Eingriff stehenden oberen Abschnitt (32) angeordnet ist und der obere und der zweite Abschnitt ein Gehäuse (30) begrenzen; und
- einer mit dem Gehäuse in Fluidverbindung stehenden Pumpe (40).

5. Vorrichtung (10) nach Anspruch 1 oder 4, bei der der Deckel (31) ein sich in den Behälter (20) erstreckendes Rohr (50) aufweist.

6. Vorrichtung (10) nach Anspruch 5, bei der das Rohr (50) mindestens einen Rührflügel (58A) an dem sich in den Behälter (20) erstreckenden Ende des Rohrs aufweist.

7. Vorrichtung (10) nach Anspruch 1 oder 4, bei der die Pumpe (40) einen oder mehrere Anschläge aufweist, um eine vorgegebene Flüssigkeitsmenge in die Pumpe zu saugen.

8. Vorrichtung (10) zur Verarbeitung einer Feststoffe enthaltenden Flüssigkeit, mit:
- einer zum Entfernen von Feststoffen aus einer Feststoffe enthaltenden Flüssigkeit geeigneten porösen Anordnung (35); und
- einem die poröse Anordnung in einer Innenkammer abstützenden Gehäuse (30),
wobei das Gehäuses einen die poröse Anordnung lösbar aufnehmenden ersten Abschnitt (32) und einen die poröse Anordnung zurückhaltenden zweiten Abschnitt (41) aufweist, wobei der erste Abschnitt einen der porösen Anordnung zugewandten Sitz (39) hat und der Sitz eine vorgegebene hydraulische Beziehung zwischen dem Gehäuse und der porösen Anordnung definiert.

9. Vorrichtung (10) nach Anspruch 8, bei der der zweite Abschnitt (41) mindestens eine mit der porösen Anordnung in Eingriff stehende Nase hat.

10. Vorrichtung (10) nach Anspruch 8, bei der der erste Abschnitt (32) einen gezahnten Abschnitt (63) zum Freigeben des zweiten Abschnitts aufweist.

11. Vorrichtung (10) nach Anspruch 8, bei der die vorgegebene hydraulische Beziehung durch eine oder mehrere Strukturen definiert wird, die so konfiguriert sind, dass sie die Oberflächenspannung des unteren Abschnitts bezüglich der porösen Anordnung verringern.

12. Vorrichtung (10) nach Anspruch 8, bei der der erste Abschnitt (32) eine oder mehrere Strukturen (60) aufweist, die so konfiguriert sind, dass sie die Fähigkeit des Zurückhaltens der porösen Anordnung im ersten Abschnitt mindern.

13. Vorrichtung (10) nach Anspruch 8, bei der der zweite Abschnitt (41) eine oder mehrere Strukturen aufweist, die so konfiguriert sind, dass sie die Fähigkeit des Zurückhaltens der porösen Anordnung im zweiten Abschnitt erhöhen.

14. Vorrichtung (10) nach Anspruch 8, ferner aufweisend einen Kanal (70) um den Sitz.

15. Vorrichtung (10) nach Anspruch 14, ferner aufweisend ein im Kanal positioniertes elastisches Element.

16. Vorrichtung (10) nach Anspruch 15, bei der das elastische Element ein O-Ring (73) oder eine Klappe (72) (74) ist.

17. Vorrichtung (10) nach Anspruch 8, bei der der Sitz (39) eine vorgegebene Oberflächenstruktur aufweist.

18. Vorrichtung (10) nach Anspruch 17, bei der der Sitz (39) eine sich radial von einem zentralen Kern aus erstreckende Neigung aufweist.

19. Vorrichtung (10) nach Anspruch 17, bei der der Sitz (39) ferner eine mit der porösen Anordnung in Eingriff stehende Klappe aufweist.

20. Vorrichtung (10) nach Anspruch 17, bei der die Oberflächenstruktur Vorsprünge (76) in einem vorgegebenen Muster aufweist.

21. Vorrichtung (10) nach Anspruch 20, bei der das Muster ein Gitter ist.

22. Vorrichtung (10) nach Anspruch 20, bei der das Muster ein Zifferblatt ist.

23. Vorrichtung (10) zur Verarbeitung einer Feststoffe enthaltenden Flüssigkeit, mit:
- einem Behälter (20) mit einem mit dem Behälter fest in Eingriff stehenden Deckel (31);
- einer porösen Anordnung (33) in einem Gehäuse (30), wobei das Gehäuse einen mit dem Deckel in Eingriff stehenden ersten Abschnitt (32) aufweist, der erste Abschnitt ein sich in den Behälter erstreckendes Rohr (50) hat, und wobei der erste Abschnitt relativ drehbar mit dem Deckel in Eingriff steht, so dass der erste Abschnitt die poröse Anordnung lösbar aufnimmt; und
- einer Pumpe (40), wobei die Pumpe einen Abschnitt aufweist, der mit einem zweiten Abschnitt (41) des Gehäuses in Eingriff gebracht zu werden vermag;
wobei das Gehäuse eine Trennung zwischen dem ersten und zweiten Abschnitt zu bilden und der zweite Abschnitt die poröse Anordnung zurückzuhalten vermag.

24. Vorrichtung (10) nach einem der Ansprüche 1 bis 23, ferner aufweisend einen Motor zum Rühren der Flüssigkeit durch relative Rotation zwischen dem Behälter und dem Gehäuse.

25. Verfahren zum Trennen von Feststoffen von einer Flüssigkeit, bei dem:
- Feststoffe enthaltende Flüssigkeit durch eine zum Entfernen von Feststoffen aus der Flüssigkeit geeignete poröse Anordnung geschickt wird, wobei die Flüssigkeit aus einem Behälter herauskommt, die poröse Anordnung in einem ersten Abschnitt eines Gehäuses aufgenommen wird, der erste Abschnitt sich relativ zum Behälter dreht und sich zur Fluidverbindung mit der Flüssigkeit in den Behälter erstreckt; und
- ein zweiter Abschnitt des Gehäuses von dem ersten Abschnitt getrennt wird, wobei der Trennschritt das Zurückhalten der porösen Anordnung in dem zweiten Abschnitt des Gehäuses beinhaltet.

26. Verfahren nach Anspruch 25, ferner umfassend das Rühren der Flüssigkeit im Behälter mittels eines am sich in den Behälter erstreckenden ersten Abschnitt befestigten Flügels.

## Revendications

1. Dispositif pour traiter un liquide contenant une substance particulaire, comprenant:
un récipient (20) comprenant un capuchon (31) en prise de manière fixe avec ledit récipient;
un agencement poreux (33) situé dans un boîtier (30), ledit boîtier comprenant une première partie (32) montée à rotation sur ledit capuchon; et
une pompe (40) montée sur une seconde partie (41) dudit boîtier;
ledit boîtier séparant ladite première partie et ladite seconde partie, et ladite seconde partie retenant ledit agencement poreux.

2. Dispositif (10) selon la revendication 1, dans lequel ladite première partie (32) est montée sur ledit capuchon avec un joint d'étanchéité aux liquides.

3. Dispositif (10) selon la revendication 2, dans lequel ledit joint d'étanchéité aux liquides n'est pas étanche aux fluides.

4. Dispositif (10) pour séparer une substance particulaire d'un liquide, comprenant:
un récipient (20) comprenant un capuchon (31), ledit capuchon comprenant une première partie (22) en prise de manière fixe avec ledit récipient, une seconde partie (71) montée à rotation sur ladite première partie;
un agencement poreux (35) en communication fluidique avec le récipient et apte à éliminer une substance particulaire d'un liquide, ledit agencement poreux étant positionné dans une partie supérieure (32) montée de façon amovible sur la seconde partie, ladite partie supérieure et ladite seconde partie définissant un boîtier (30); et
une pompe (40) en communication fluidique avec ledit boîtier.

5. Dispositif (10) selon la revendication 1 ou 4, dans lequel ledit capuchon (31) comprend un tube (50) qui s'étend dans ledit récipient (20).

6. Dispositif (10) selon la revendication 5, dans lequel ledit tube (50) comprend au moins une ailette d'agitation (58A) située sur l'extrémité du tube, qui pénètre dans ledit récipient (20).

7. Dispositif (10) selon la revendication 1 ou 4, dans lequel ladite pompe (40) comprend plusieurs dispositifs d'arrêt pour introduire une quantité prédéterminée de liquide dans la pompe.

8. Dispositif (10) pour traiter un liquide contenant une substance particulaire comprenant:
un agencement poreux (35) convenant pour retirer une matière particulaire d'un liquide contenant des particules; et
un boîtier (30) supportant ledit agencement poreux dans une chambre intérieure, le boîtier incluant une première partie (30) recevant de façon amovible ledit agencement poreux, et une seconde partie (41) retenant ledit agencement poreux, ledit boîtier séparant lesdites première et seconde parties, ladite première partie possédant un siège (39) situé en regard dudit agencement poreux, ledit siège définissant une relation hydraulique prédéterminée entre ledit boîtier et ledit agencement poreux.

9. Dispositif (10) selon la revendication 8, dans lequel ladite seconde partie (41) comprend au moins un moyeu en prise avec l'agencement poreux.

10. Dispositif (10) selon la revendication 8, dans lequel ladite première partie (32) comprend une partie crénelée (63) pour le désengagement de la seconde partie.

11. Dispositif (10) selon la revendication 8, dans lequel ladite relation hydraulique prédéterminée est définie par une ou plusieurs structures configurées de manière à réduire la tension de surface de ladite partie inférieure par rapport audit agencement poreux.

12. Dispositif (10) selon la revendication 8, dans lequel ladite première partie (32) comprend une ou plusieurs structures (60) configurées de manière à réduire la capacité de retenue de l'agencement poreux dans ladite première partie.

13. Dispositif (10) selon la revendication 8, dans lequel ladite seconde partie (41) comprend une ou plusieurs structures configurées de manière à accroître la capacité de retenue de l'agencement poreux dans ladite seconde partie.

14. Dispositif (10) selon la revendication 8, comprenant en outre un canal (70) autour dudit siège.

15. Dispositif (10) selon la revendication 14, comprenant en outre un élément élastique positionné dans ledit canal.

16. Dispositif (10) selon la revendication 15, dans lequel ledit élément élastique est un joint torique (73) ou un rabat (72) (74) .

17. Dispositif (10) selon la revendication 8, dans lequel ledit siège (32) comprend une texture de surface prédéterminée.

18. Dispositif (10) selon la revendication 17, dans lequel ledit siège (39) comprend une pente qui s'étend radialement à partir d'un noyau central.

19. Dispositif (10) selon la revendication 17, dans lequel ledit siège (39) comprend en outre un rabat en prise avec l'agencement poreux.

20. Dispositif (10) selon la revendication 17, dans lequel la texture de surface comporte des parties saillantes (76) disposées selon un motif prédéterminé.

21. Dispositif (10) selon la revendication 20, dans lequel le réseau est une grille.

22. Dispositif (10) selon la revendication 20, dans lequel le réseau est un cadran.

23. Dispositif (10) pour traiter un liquide contenant une substance particulaire, comprenant:
un récipient (20) incluant un capuchon (31) en prise de manière fixe avec ledit récipient;
un agencement poreux (33) situé dans un boîtier (30), ledit boîtier comprenant une première partie (32) en prise avec ledit capuchon, ladite première partie possédant un tube (50) qui s'étend dans ledit récipient, ladite première partie montée à rotation sur ledit capuchon, ladite première partie logeant de façon amovible ledit agencement poreux; et
une pompe (40) comprenant une partie apte à venir en prise avec une seconde partie (41) dudit boîtier;
ledit boîtier étant apte à séparer ladite première partie et ladite seconde partie, et ladite seconde partie étant apte à retenir ledit agencement poreux.

24. Dispositif (10) selon l'une quelconque des revendications 1 à 23, comprenant en outre:
un moteur agitant le liquide sous l'effet d'une rotation relative entre ledit récipient et ledit boîtier.

25. Procédé pour séparer une substance particulaire d'un liquide, consistant à:
faire passer un liquide contenant des particules dans un agencement poreux convenant pour le retrait de la substance particulaire à partir dudit liquide, ledit liquide circulant à partir de l'intérieur d'un récipient, ledit agencement poreux étant logé dans une première partie d'un boîtier, ladite première partie étant montée à rotation par rapport audit récipient et s'étendant dans ledit récipient pour une communication fluidique avec le liquide; et
séparer une seconde partie dudit boîtier de ladite première partie, ladite étape de séparation incluant la retenue dudit agencement poreux dans ladite seconde partie dudit boîtier.

26. Procédé selon la revendication 25, comprenant en outre une étape consistant à agiter le liquide dans ledit récipient avec une ailette fixée à ladite première partie s'étendant dans ledit récipient.
